# EUROPEAN PATENT SPECIFICATION

(11) **EP 2 324 007 B1**
(45) Date of publication and mention of the grant of the patent: **15.08.2012**
(21) Application number: 09782447.8
(22) Date of filing: 01.09.2009
(51) Int. Cl.: C07D 401/04, C07D 407/14, A61K 31/4196, A61P 25/00

(54) **TRIAZOLE DERIVATIVES AND THEIR USE AS NICOTINIC ACETYLCHOLINE RECEPTOR MODULATORS**
TRIAZOLDERIVATE UND IHRE VERWENDUNG ALS MODULATOREN VON NICOTINISCHEN ACETYLCHOLINREZEPTOREN
DÉRIVÉS DE TRIAZOLE ET LEUR UTILISATION EN TANT QUE MODULATEURS DE RÉCEPTEUR NICOTINIQUE D ACÉTYLCHOLINE

(30) Priority: 02.09.2008 DK 200801220; 04.09.2008 US 94136
(43) Date of publication of application: 25.05.2011
(73) Proprietor: NeuroSearch A/S, 2750 Ballerup (DK)
(72) Inventor: DAHL, Bjarne, H., DK-3540 Lynge (DK); OLSEN, Gunnar, M., DK-2765 Smørum (DK); CHRISTENSEN, Jeppe, Kejser, DK-2750 Ballerup (DK); PETERS, Dan, DK-2750 Ballerup (DK)
(74) Representative: Abildgren, Michael Padkjaer
(86) International application number: PCT/EP2009/061262
(87) International publication number: WO 2010/026134

(56) References cited:
- WO-A-01/87288
- WO-A-2007/131991
- WO-A-2007/138033
- WO-A-2009/019278
- US-A- 4 474 599
- US-B1- 6 303 638
- ROPPE JEFFREY ET AL: "Discovery of Novel Heteroarylazoles That Are Metabotropic Glutamate Subtype 5 Receptor Antagonists with Anxiolytic Activity" JOURNAL OF MEDICINAL CHEMISTRY, AMERICAN CHEMICAL SOCIETY, WASHINGTON, US, vol. 47, no. 19, 14 August 2004 (2004-08-14), pages 4645-4648, XP002518935 ISSN: 0022-2623
- DATABASE BEILSTEIN BEILSTEIN INSTITUTE FOR ORGANIC CHEMISTRY, FRANKFURT-MAIN, DE; XP002551530 Database accession no. BRN: 11176688 & CHEMISTRY LETTERS, vol. 36, no. 9, 2007, pages 1142-1143,
- DATABASE BEILSTEIN BEILSTEIN INSTITUTE FOR ORGANIC CHEMISTRY, FRANKFURT-MAIN, DE; XP002551531 Database accession no. BRN: 7875444 & JOURNAL OF HETEROCYCLIC CHEMISTRY, vol. 34, no. 6, 1997, pages 1645-1650,
- SCHWEINFURTH D ET AL: "1,3-Dipolar cycloaddition of alkylnes to azides. Construction of operationally functional metal responsive fluorophores" CHEM. COMMUN., 28 March 2008 (2008-03-28), pages 2203-2205, XP002551526
- PARK I S ET AL: "Heterogeneous copper catalyst for the cycloaddition of azides and alkynes without additves under ambient conditions" ORGANIC LETTERS, vol. 10, no. 3, 1 September 2008 (2008-09-01), pages 497-500, XP002551527
- MOORHOUSE A D ET AL: "Microwave enhancement of a "one-pot" tandem azidation-"click" cycloaddition of anilines" SYNLETT, no. 14, 31 July 2008 (2008-07-31), pages 2089-2092, XP002551528
- BANKS R E ET AL: "Studies in azide chemistry. Part V. Synthesis of 4-azido-2,3,5,6-tetarfluoro-, 4-azido-3-chloro-2,5,6-trifluoro-, 4-azido-3,5-dichloro-2,6-difluoro-pyridine , and some thermal reactions of the tetrafluoro-compound" J. CHEM. SOC., PERKIN TRANS. 1, 1972, pages 2964-2970, XP002551529
- DATABASE CAPLUS [Online] CHEMICAL ABSTRACTS SERVICE, COLUMBUS, OHIO, US; XP002551532 retrieved from STN accession no. 2004:858218 Database accession no. 142:280129 & CHINESE JOURNAL OF CHEMISTRY, vol. 22, no. 10, 2004, pages 1183-1186,

## Description

### TECHNICAL FIELD

This invention relates to novel triazole derivatives, which are found to be modulators of the nicotinic acetylcholine receptors. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exerts its biological effect via two types of cholinergic receptors, the muscarinic Acetyl Choline Receptors (mAChR) and the nicotinic Acetyl Choline Receptors (nAChR).

Nicotinic acetylcholine receptors (nAChRs) are pentameric ligand gated ion channels and widely distributed throughout the central (CNS) and peripheral (PNS) nervous systems. At least 12 subunit proteins, i.e. α2-α10 and β2-β4, have been identified in neuronal tissue. These subunits provide for a great variety of homomeric and heteromeric combinations that account for the diverse receptor subtypes. For example, the predominant receptor that is responsible for high affinity binding of nicotine in brain tissue has composition α4β2, while another major population of receptors is comprised of the homomeric α7.

Discovery of the important role played by nAChRs in several CNS disorders has called attention to these membrane proteins and to ligands able to modulate their functions. The existence of different subtypes at multiple levels has complicated the understanding of this receptor's physiological role, but at the same time has increased the efforts to discover selective compounds in order to improve the pharmacological characterization of this kind of receptor and to make safer the possible therapeutic use of its modulators.

WO 2005/090333 describes the preparation of triazolyl arylbenzamides useful as inhibitors of cytokines. WO 2007/025089 describes the preparation of macrolide clarithromycin triazole glycosides as anti-infective, anti-proliferative, anti-inflammatory and prokinetic agents. WO 2008/003770 describes the preparation of phenyltriazoles and related compounds as anti-tumour agents. Modulators of nicotinic acethylcholine receptors are disclosed in WO 2007/138033, WO01/87288 and US 6,303,638. However, the triazole derivatives of the present invention have never been disclosed.

### SUMMARY OF THE INVENTION

The present invention is devoted to the provision modulators of the nicotinic receptors, which modulators are useful for the treatment of diseases or disorders related to the nicotinic acetylcholine receptor (nAChR).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and abuse liability and withdrawal symptoms caused by the termination of abuse of chemical substances, in particular nicotine.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides a triazole derivative of Formula I 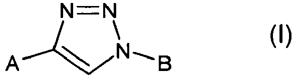 a stereoisomer or a mixture of its stereoisomers, a prodrug, or a pharmaceutically acceptable addition salt thereof, wherein
one of A and B represents a pyridinyl group optionally substituted one or more times with a substituent selected from halo, trifluoromethyl, trifluoromethoxy, cyano, nitro and alkoxy; and
the other one of A and B represents a pyridinyl group optionally substituted one or more times with a substituent selected from halo, trifluoromethyl, trifluoromethoxy, cyano and nitro.

In a second aspect the invention provides pharmaceutical compositions comprising a therapeutically effective amount of the triazole derivative of the invention, or a pharmaceutically acceptable addition salt thereof, together with at least one pharmaceutically acceptable carrier or diluent.

Viewed from another aspect the invention relates to the use of the triazole derivative of the invention, or a pharmaceutically acceptable addition salt thereof, for the manufacture of pharmaceutical compositions/medicaments for the treatment, prevention or alleviation of a disease or a disorder or a condition of a mammal, including a human, which disease, disorder or condition is responsive to modulation of cholinergic receptors.

Other objects of the invention will be apparent to the person skilled in the art from the following detailed description and examples.

### DETAILED DISCLOSURE OF THE INVENTION

### Triazole derivatives

In its first aspect the invention provides a triazole derivative of Formula I 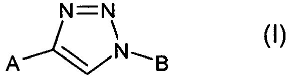 a stereoisomer or a mixture of its stereoisomers, a prodrug, or a pharmaceutically acceptable addition salt thereof, wherein one of A and B represents a pyridinyl group optionally substituted one or more times with a substituent selected from halo, trifluoromethyl, trifluoromethoxy, cyano, nitro and alkoxy; and the other one of A and B represents a pyridinyl group optionally substituted one or more times with a substituent selected from halo, trifluoromethyl, trifluoromethoxy, cyano and nitro.

In another more preferred embodiment A represents pyridinyl, optionally substituted one or more times with a substituent selected from halo, trifluoromethyl, trifluoromethoxy, cyano, nitro and alkoxy.

In a third more preferred embodiment A represents pyridinyl, optionally substituted one or more times with a substituent selected from halo, trifluoromethyl, trifluoromethoxy, cyano and nitro.

In a fourth more preferred embodiment A represents pyridinyl substituted with halo, trifluoromethyl, trifluoromethoxy, cyano, nitro or alkoxy.

In a fifth more preferred embodiment A represents pyridinyl substituted with halo, trifluoromethyl, trifluoromethoxy, cyano or nitro.

In a sixth more preferred embodiment A represents pyridinyl substituted with halo, and in particular fluoro or chloro, or with alkoxy, and in particular methoxy.

In a seventh more preferred embodiment A represents pyridinyl substituted with halo, and in particular fluoro or chloro.

In an eight more preferred embodiment A represents pyridinyl substituted with alkoxy, and in particular methoxy.

In a ninth more preferred embodiment A represents pyridinyl.

In a sixteenth more preferred embodiment B represents a pyridinyl group, optionally substituted one or more times with a substituent selected from halo, trifluoromethyl, trifluoromethoxy, cyano and nitro.

In a seventeenth more preferred embodiment B represents a pyridinyl group substituted with halo, trifluoromethyl or trifluoromethoxy.

In an eighteenth more preferred embodiment B represents a pyridinyl group substituted with halo, and in particular chloro.

In a nineteenth more preferred embodiment B represents a pyridinyl group, optionally substituted with halo.

In a twentieth more preferred embodiment B represents a pyridinyl group.

In another more preferred embodiment both of A and B represent a pyridinyl group, optionally substituted one or more times with a substituent selected from halo, trifluoromethyl, trifluoromethoxy, cyano, nitro and alkoxy.

In a third more preferred embodiment both of A and B represent a pyridinyl group, optionally substituted one or more times with a substituent selected from halo, trifluoromethyl, trifluoromethoxy, cyano and nitro.

In a fourth more preferred embodiment both of A and B represent a pyridinyl group substituted with halo, and in particular fluoro, or with alkoxy, and in particular methoxy.

In a fifth more preferred embodiment both of A and B represent a pyridinyl group substituted with halo, and in particular fluoro.

In a sixth more preferred embodiment both of A and B represent a pyridinyl group substituted with alkoxy, and in particular methoxy.

In a seventh more preferred embodiment both of A and B represent a pyridinyl group.

In a most preferred embodiment the compound of the invention is
3-(1-Pyridin-3-yl-1*H*-[1,2,3]triazol-4-yl)-pyridine;
3-Fluoro-5-(1-pyridin-3-yl-1H-[1,2,3]triazol-4-yl)-pyridine;
2-Fluoro-5-(1-pyridin-3-yl-1H-[1,2,3]triazol-4-yl)-pyridine;
2-methoxy-5-(1-pyridin-3-yl-1H-[1,2,3]triazol-4-yl)-pyridine;
3-Fluoro-5-(4-pyridin-3-yl-[1,2,3]triazol-1-yl)-pyridine;
2-Fluoro-5-(4-pyridin-3-yl-[1,2,3]triazol-1-yl)-pyridine; or
3-Bromo-5-(4-pyridin-3-yl-[1,2,3]triazol-1-yl)-pyridine;
any of its isomers or any mixture of isomers, or a pharmaceutically acceptable addition salt thereof.

Any combination of two or more of the embodiments described herein is considered within the scope of the present invention.

### Pharmaceutically acceptable salts

The triazole derivative of the invention may be provided in any form suitable for the intended administration. Suitable forms include pharmaceutically (i.e. physiologically) acceptable salts, and pre- or prodrug forms of the compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzene-sulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a compound of the invention include alkali metal salts, such as the sodium salt of a compound of the invention containing a carboxy group.

In the context of this invention the "onium salts" of N-containing compounds may also be contemplated as pharmaceutically acceptable salts. Preferred "onium salts" include the alkyl-onium salts, the cycloalkyl-onium salts, and the cycloalkylalkyl-onium salts.

### Steric isomers

It will be appreciated by those skilled in the art that the compounds of the present invention may exist in different stereoisomeric forms, including enantiomers, diastereomers, as well as geometric isomers (cis-trans isomers). The invention includes all such stereoisomers and any mixtures thereof including racemic mixtures.

Racemic forms can be resolved into the optical antipodes by known methods and techniques. One way of separating the enantiomeric compounds (including enantiomeric intermediates) is by use of an optically active amine, and liberating the diastereomeric, resolved salt by treatment with an acid. Another method for resolving racemates into the optical antipodes is based upon chromatography on an optical active matrix. Racemic compounds of the present invention can thus be resolved into their optical antipodes, e.g., by fractional crystallisation of D- or L- (tartrates, mandelates or camphorsulphonate) salts for example.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials or intermediates.

### Methods of producing triazole derivatives

The triazole derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological activity

The present invention is devoted to the provision modulators of the nicotinic receptors, which modulators are useful for the treatment of diseases or disorders related to the nicotinic acetylcholine receptor (nAChR). Preferred compounds of the invention show a positive allosteric modulation of the nicotinic acetylcholine α4β2 receptor subtypes.

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and abuse liability and withdrawal symptoms caused by the termination of abuse of chemical substances, in particular nicotine.

In a preferred embodiment the disease, disorder or condition relates to the central nervous system.

The compounds of the invention may also be useful as diagnostic tools or monitoring agents in various diagnostic methods, and in particular for *in vivo* receptor imaging (neuroimaging), and they may be used in labelled or unlabelled form.

In another preferred embodiment the disease, disorder or condition is a cognitive disorder, learning deficit, memory deficits and dysfunction, Down's syndrome, Alzheimer's disease, attention deficit, attention deficit hyperactivity disorder (ADHD), Tourette's syndrome, psychosis, depression, bipolar disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, amyotrophic lateral sclerosis (ALS), anxiety, non-OCD anxiety disorders, convulsive disorders, convulsions, epilepsy, neurodegenerative disorders, transient anoxia, induced neuro-degeneration, neuropathy, diabetic neuropathy, peripheral dyslexia, tardive dyskinesia, hyperkinesia, pain, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to postherpetic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or abuse liability and withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention are used for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to postherpetic neuralgia, or to peripheral nerve injury.

In another more preferred embodiment the compounds of the invention are used for the treatment, prevention or alleviation of smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a third more preferred embodiment the compounds of the invention are used for the treatment, prevention or alleviation of a neurodegenerative disorder, transient anoxia, or induced neuro-degeneration.

In a fourth more preferred embodiment the compounds of the invention are used for the treatment, prevention or alleviation of an inflammatory disorder, inflammatory skin disorder, acne, rosacea, Crohn's disease, inflammatory bowel disease, ulcerative colitis, or diarrhoea.

In a fifth more preferred embodiment the compounds of the invention are used for the treatment, prevention or alleviation of diabetic neuropathy, schizophrenia, cognitive or attention deficits related to schizophrenia, or depression.

In a sixth more preferred embodiment the compounds of the invention are used for the treatment, prevention or alleviation of pain, in particular neuropathic pain, diabetic neuropathy, schizophrenia and cognitive or attention deficits related to schizophrenia, depression, and for assisting in obtaining smoking cessation.

In a seventh more preferred embodiment the compounds of the invention are used the treatment of abuse liability and withdrawal symptoms caused by termination of use of addictive substances, in particular nicotine containing products such as tobacco, opioids such as heroin, cocaine and morphine, cannabis, benzodiazepines, benzodiazepine-like drugs, and alcohol.

In an eighth more preferred embodiment the compounds of the invention are used for the treatment of anxiety, cognitive disorders, learning deficit, memory deficits and dysfunction, Down's syndrome, Alzheimer's disease, attention deficit, attention deficit hyperactivity disorder (ADHD), Parkinson's disease, Huntington's disease, Amyotrophic Lateral Sclerosis, Gilles de la Tourette's syndrome, psychosis, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, peripheral neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In a ninth more preferred embodiment the compounds of the invention are used for the treatment of cognitive disorders, psychosis, schizophrenia and/or depression.

In a tenth more preferred embodiment the compounds of the invention are used for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In an eleventh more preferred embodiment the compounds of the invention are used for the treatment of endocrine disorders, such as thyrotoxicosis, pheochromocytoma, hypertension and arrhythmias.

In a twelfth more preferred embodiment the compounds of the invention are used for the treatment of neurodegenerative disorders, including transient anoxia and induced neuro-degeneration.

In a thirteenth more preferred embodiment the compounds of the invention are used for the treatment of inflammatory diseases, disorders, or conditions, including inflammatory skin disorders such as acne and rosacea, Crohn's disease, inflammatory bowel disease, ulcerative colitis, and diarrhoea.

In a fourteenth more preferred embodiment the compounds of the invention are used for the treatment of pain, mild, moderate or severe pain, or pain of acute, chronic or recurrent character, as well as pain caused by migraine, postoperative pain, and phantom limb pain. The pain may in particular be neuropathic pain, chronic headache, central pain, pain related to diabetic neuropathy, to postherpetic neuralgia, or to peripheral nerve injury.

Finally, in a most preferred embodiment, the compounds of the invention may be useful for the treatment of depression, cognition, dementia, obesity, or associated with abuse liability and withdrawal symptoms caused by nicotine addiction.

In this context "treatment" covers treatment, prevention, prophylactics and alleviation of abuse liability and withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

### Pharmaceutical compositions

In another aspect the invention provides novel pharmaceutical compositions comprising a therapeutically effective amount of a triazole derivative of the invention, a stereoisomer or a mixture of its stereoisomers, a prodrug, or a pharmaceutically acceptable addition salt thereof.

While a compound of the invention for use in therapy may be administered in the form of the raw compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the triazole derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by the skilled person by use of standard methods and conventional techniques appropriate to the desired formulation. When desired, compositions adapted to give sustained release of the active ingredient may be employed.

In a preferred embodiment, when the pharmaceutical composition of the invention is intended for treating patients with abuse liability and withdrawal symptoms caused by nicotine addiction, formulations such as gums, patches, sprays, inhalers, aerosols, etc., are contemplated.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### Methods of therapy

The triazole derivatives of the present invention are valuable nicotinic and monoamine receptor modulators, and therefore useful for the treatment of a range of ailments involving cholinergic dysfunction as well as a range of disorders responsive to the action of nAChR modulators.

In another aspect the invention provides a triazole derivative of the invention for use as a medicament. In a further aspect the invention provides the use of a triazole derivative of the invention for the manufacture of a pharmaceutical composition/medicament for the treatment, prevention or alleviation of a disease or a disorder or a condition of a living animal body, including a human, which disease, disorder or condition is responsive to modulation of cholinergic receptors and/or monoamine receptors.

In the context of this invention the term "treatment" covers treatment, prevention, prophylaxis or alleviation, and the term "disease" covers illnesses, diseases, disorders and conditions related to the disease in question.

The preferred indications contemplated according to the invention are those stated above.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

A satisfactory result can, in certain instances, be obtained at a dosage as low as 0.005 mg/kg i.v. and 0.01 mg/kg p.o. The upper limit of the dosage range is about 10 mg/kg i.v. and 100 mg/kg p.o. Preferred ranges are from about 0.001 to about 1 mg/kg i.v. and from about 0.1 to about 10 mg/kg p.o.

### EXAMPLES

The invention is further illustrated with reference to the following examples, which are not intended to be in any way limiting to the scope of the invention as claimed.

### Preparatory examples

### Example 1

### 3-Trimethylsilanethylnyl-benzonitrile (Intermediate compound)

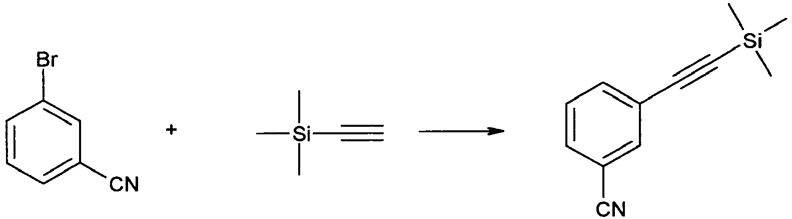

Tetrahydrofuran (50 mL) was added to a mixture of 3-bromo benzonitrile (2 g, 10.9 mmol), triphenylphosphine (0.3 g, 1.1 mmol) cuprous iodide (0.21 g, 1.1 mmol) and palladium(II) (bistriphenylphoshine)dichloride, and the mixture was degassed. Triethylamine (3.0 mL, 21. 8 mmol) was added and the mixture was stirred at room temperature under a nitrogen atmosphere for 10 min. Over 15 min. trimethylsilylacethylene (1.4 g, 14.1 mmol) was added in drops. The reaction mixture was stirred at room temperature overnight.

No starting material remained. The mixture was concentrated under reduced pressure, and the residue was added hexane (50 mL) and filtrated. The filtrate was washed with water and evaporated to a brown solid. Yield 1.9 g (89%).

### 3-Ethylnyl-benzonitrile (Intermediate compound)

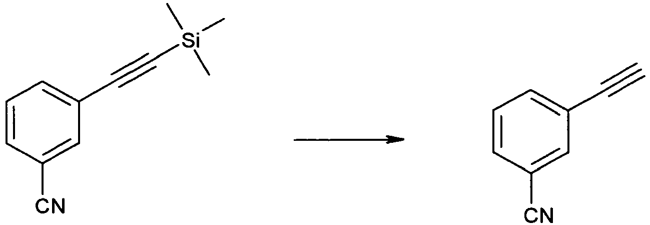

Potassium carbonate (1.3 g, 9.4 mmol) was added to a solution of 3-trimethylsilanethylnyl- benzonitrile (1.9 g, 9.4 mmol) dissolved in methanol (50 mL). The reaction mixture was stirred at room temperature for 27 hours and filtrated. The filtrate was washed with methanol and evaporated to a black gum. The residue was dissolved in ethyl acetate, the organic layer was washed with water, brine and dried over anhydrous sodium sulphate. The product was isolated by column chromatography on silica gel (height 10 cm, diameter 2 cm) eluted with 18% ethyl acetate in hexane. The product fractions were evaporated under vacuum to a brown solid. Yield 0.7 g (59%).

### Synthesis of 3-Azido-benzonitrile (Intermediate compound)

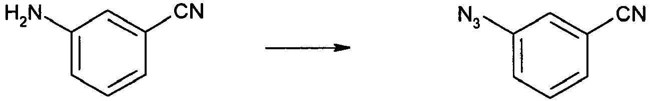

3-Amino benzonitrile (3 g, 25.1 mmol) in water (40 mL) was added conc. hydrochloric acid (10 mL), in such a rate that the reaction mixture was below 5°C. Sodium nitrite (1.9 g, 28 mmol) was added. The reaction mixture was stirred for 30 min. at 0-5°C. Sodium azide (1.8 g, 28 mmol) in water (10 mL) was added drop by drop. A solid precipitated and the reaction mixture was added diethyl ether (100 mL). The phases were separated and the water phase was extracted with diethyl ether. The organic phases were mixed and dried with anhydrous sodium sulphate and evaporated to a brown solid. The product was isolated by column chromatography on silica gel (height 20 cm, diameter 5 cm), using hexane/ethyl acetate (7/3) as eluent. Evaporation of the product fractions gave a white solid. Yield 2.1 g (58%).

### 3-azido pyridine (Intermediate compound)

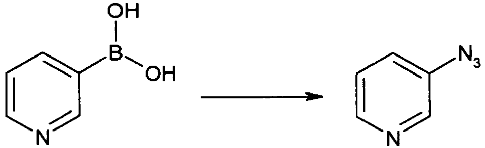

Cupric sulphate (0.13 g, 0.8 mmol) was added to a solution of pyridine-3-boronic acid in a mixture of water (10 mL) and methanol (5 mL). After 10 min. sodium azide (0.6 g, 8.9 mmol) was added. The reaction mixture was stirred at room temperature for one hour and concentrated under vacuum. The residue was dissolved in ethyl acetate and filtrated. The filtrate was washed with water, brine, dried over anhydrous sodium sulphate and filtered. The filtrate was concentrated to afford a black solid. The product was isolated by column chromatography on silica gel (60-120 mesh) (height 15 cm, diameter 3 cm) eluted with 5% ethyl acetate in hexane. The product fractions were evaporated to give an off-white solid. Yield 0.7 g (84%).

### 3-(4-Pyridin-3-yl-[1,2,31triazol-1-yl)-benzonitrile (Compound 1A) - Reference compound

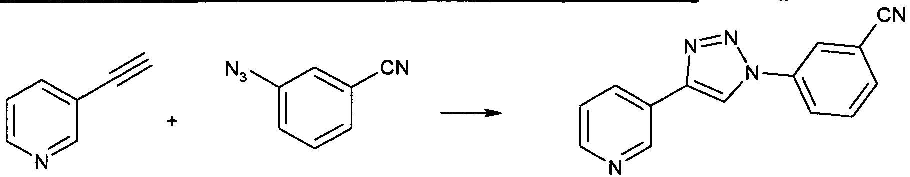

3-Ethynyl pyridine was added to a suspension of 3-azido-benzonitrile in a mixture of methanol and water. The mixture was stirred for 5 min. and L-ascorbic acid sodium salt (1.2 g, 6.2), copper sulphate (0.02 g, 0.1 mmol) and triethyl amine (2.9 mL, 20.8 mmol) were added. The reaction mixture was stirred at room temperature overnight and concentrated under vacuum. The residue was added cool water, a solid precipitated which was isolated by filtration to give a brown solid. The product was isolated by solid chromatography on silica gel (height 20 cm, diameter 4 cm) eluted with dichloromethane/methanol (9/1), and the product fractions were evaporated under vacuum to give a off white solid. Yield 1.4 g (54%). Mp. 214-216°C. LC-ESI-HRMS of [M+H]+ shows 248.0946 Da. Calc. 248.09362 Da, dev. 4 ppm.

In analogy herewith the following compounds were synthesised:
5-(4-Pyridin-3-yl-[1,2,3]triazol-1-yl)-furan-2-carboxylic acid methyl ester (Intermediate compound)
3-(1-Pyridin-3-yl-1*H*-[1,2,3]triazol-4-yl)-pyridine (Compound 1B)
   Mp.217-219°C.
3-(1-Pyridin-3-yl-1*H*-[1,2,3]triazol-4-yl)-benzonitrile(Compound 1C)
   LC-ESI-HRMS of [M+H]+ shows 248.0935 Da. Calc. 248.09362 Da, dev. -0.5 ppm.
3-(3-Cyanophenyl-1*H*-[1,2,3]triazol-4-yl)-benzonitrile (Compound 1 D)
   LC-ESI-HRMS of [M+H]+ shows 272.0935 Da. Calc. 272.093075 Da, dev. 1.6 ppm. Mp. 214.4-217.3°C.
3-[4-(2-Chloro-6-fluoro-phenyl)-[1,2,3]triazol-1-yl]-pyridine (Compound 1E)
   LC-ESI-HRMS of [M+H]+ shows 275.0494 Da. Calc. 275.049432 Da, dev. -0.1 ppm Mp. 94-95.2°C.
3-[1-(3-Chloro-phenyl)-1H-[1,2,3]triazol-4-yl]-pyridine (Compound 1F)
   LC-ESI-HRMS of [M+H]+ shows 257.05898 Da. Calc. 257.058854 Da, dev. 0.5 ppm. 170-171 °C.
2-Chloro-5-[4-(3-isocyano-phenyl)-[1,2,3]triazol-1-yl]-pyridine (Compound 1G)
   LC-ESI-HRMS of [M+H]+ shows 282.0539 Da. Calc. 282.054103 Da, dev. -0.7 ppm. Mp. 202.2-203.6°C.
3-[4-(6-Chloro-pyridin-3-yl)-[1,2,3]triazol-1-yl]-benzonitrile (Compound 1H)
   LC-ESI-HRMS of [M+H]+ shows 282.0536 Da. Calc. 282.054103 Da, dev. -1.8 ppm. Mp. 219.3-220°C.
3-[1-(2-Chloro-6-fluoro-phenyl)-1H-[1,2,3]triazol-4-yl]-pyridine (Compound 1I)
   LC-ESI-HRMS of [M+H]+ shows 275.04879 Da. Calc. 275.049432 Da, dev. -2.3 ppm. Mp. 94.5-97.1°C.
3-[4-(2-Chloro-pyridin-3-yl)-[1,2,3]triazol-1-yl]-benzonitrile (Compound 1J)
   LC-ESI-HRMS of [M+H]+ shows 282.053 Da. Calc. 282.054103 Da, dev. -3.9 ppm. Mp. 181-182.6°C.
2-Chloro-3-[4-(3-isocyano-phenyl)-[1,2,3]triazol-1-yl]-pyridine(Compound 1K)
   LC-ESI-HRMS of [M+H]+ shows 282.05403 Da. Calc. 282.054103 Da, dev. -0.3 ppm. Mp. 184-185°C.
3-Fluoro-5-(1-pyridin-3-yl-1H-[1,2,3]triazol-4-yl)-pyridine (Compound 1L)
   Mp. 190.2-191.6.
2-Fluoro-5-(1-pyridin-3-yl-1H-[1,2,3]triazol-4-yl)-pyridine (Compound 1M)
   Mp. 187-188.7°C.
2-Methoxy-5-(1-pyridin-3-yl-1H-[1,2,3]triazol-4-yl)-pyridine (Compound 1N)
   Mp. 205.6-206.9°C.
3-Fluoro-5-(4-pyridin-3-yl-[1,2,3]triazol-1-yl)-pyridine (Compound 1O)
   Mp. 190.6-191.7°C.
2-Fluoro-5-(4-pyridin-3-yl-[1,2,3]triazol-1-yl)-pyridine (Compound 1P)
   Mp. 221.3-222.6°C.
3-Bromo-5-(4-pyridin-3-yl-[1,2,3]triazol-1-yl)-pyridine (Compound 1Q)
   Mp. 226.2-227.8°C.
3-[1-(3-Fluoro-phenyl)-1H-[1,2,3]triazol-4-yl]-pyridine (Compound 1R)
   Mp. 156.3-158.2°C.
3-[1-(4-Fluoro-phenyl)-1H-[1,2,3]triazol-4-yl]-pyridine (Compound 1S)
   Mp. 179.9-190.9°C.
   Compounds 1C - 1K, 1R and 1S are reference compound

### Example 2

### 5-(4-Pyridin-3-yl-[1,2,3]triazol-1-yl)-furan-2-carboxylic acid (Intermediate compound)

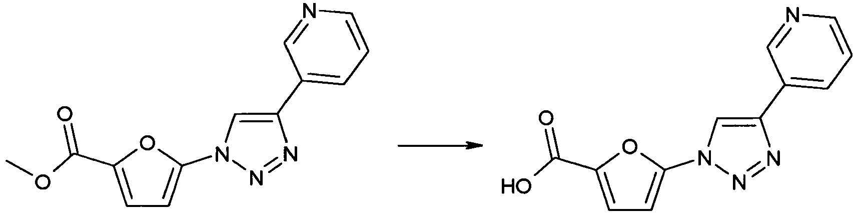

Potassium hydroxide (0.15 g, 2.8 mmol) was added to a solution of 5-(4-pyridin-3-yl-[1,2,3]triazol-1-yl)-furan-2-carboxylic acid methyl ester (0.75 g, 2.8 mmol) in tetrahydrofuran/water (1/1) at 0°C. The reaction mixture was stirred at room temperature for 2 hours. Hydrochloric acid (1.5 N) was added until the pH was between 5 and 6, and the solid formed was isolated by filtration. The white solid was dried in vacuum. Yield 0.66 g (92%).

### 5-(4-Pyridin-3-yl-[1,2,3]triazol-1-yl)-furan-2-carboxylic acid amide (Intermediate compound)

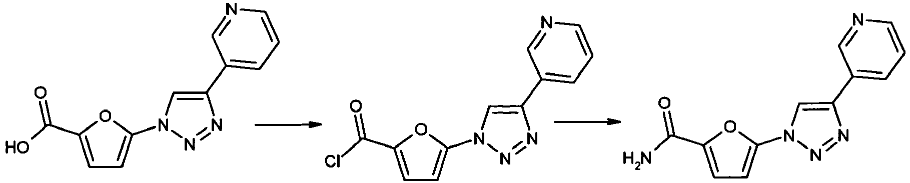

5-(4-Pyridin-3-yl-[1,2,3]triazol-1-yl)-furan-2-carboxylic acid (0.65 g, 2.5 mmol) was added to oxalyl chloride (10 mL, 118 mmol), and the mixture was added N,N-dimethylformamide (20 µL, 0.25 mmol). The reaction mixture was stirred at 45°C overnight.

The reaction mixture was concentrated to remove oxalyl chloride and the residue was dissolved in tetrahydrofuran. Aqueous solution of ammonia (25%) was added, and the reaction mixture was stirred at room temperature for overnight. Water was added to the reaction mixture, a solid precipitated and this was isolated by filtration, washed with water, and dried in vacuum to give an off-white solid. Yield 0.61 g (95%).

### 5-(4-Pyridin-3-yl-[1,2,3]triazol-1-yl)-furan-2-carbonitrile (Compound 2A) Reference

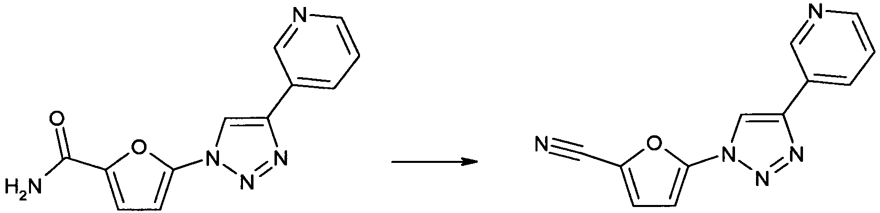

Pyridine (0.38 mL, 4.7 mmol) was added to a suspension of 5-(4-pyridin-3-yl-[1,2,3]triazol-1-yl)-furan-2-carboxylic acid amide (0.6 g, 2.4 mmol) in dichloromethane (20 mL), and the mixture was cooled to 0°C. Trifluoromethanesulfonic anhydride (1 g, 5.9 mmol) was added, and the reaction mixture was stirred at room temperature overnight.

The reaction mixture was added a mixture of ice/water and extracted with dichloromethane (150 mL, 3 times). The organic phase was washed with water (50 mL), brine (50 mL), dried over anhydrous sodium sulphate and concentrated under vacuum to give a brown gum. The product was isolated by column chromatography over silica gel (height 7 cm, diameter 2 cm) eluted with chloroform/methanol (9/1). The product fractions were evaporated to give a brown solid. Yield 0.023 g (4%). LC-ESI-HRMS of [M+H]+ shows 238.0726 Da. Calc. 238.07234 Da, dev. 1.1 ppm.

### Example 3

### Biological activity

### Characterization of hα4β2 positive allosteric modulators using FLIPR

This experiment shows the modulating activity of compounds representative of the invention (i.e. Compounds 1 and 2) to positively modulate the response induced by a range of concentrations of nicotine (0.1 nM-100 mM) in human HEK-293 cells stably expressing the human nicotinic acetylcholine receptor subtype a4b2. The modulator activity is determined as a fluorescence-based assay using a Fluorometric Imaging Plate Reader (FLIPR) as described below in more detail.

Full concentration-response curves of nicotine +/- test compound are generated and delta pEC₅₀ values are calculated based on EC₅₀ values for nicotine +/- test compound. EC₅₀ values (Effective Concentration 50%) represent the concentration of the nicotine, at which the nicotine-induced response is 50% of the maximal nicotine-induced response.

Preferred compounds of the invention show an activity determined as delta pEC₅₀ values above 1 in the presence of 10 µM of the test compound compared to nicotine alone.

The results of this experiment are presented in Table 1 below.

**Table 1**

| FLIPR nAChR a4b2 positive allosteric modulator activity | |
|---|---|
| Compound | Delta pEC₅₀ (mM) |
| 1 | 1.6 |
| 2 | 1.2 |

## Claims

1. A triazole derivative represented by Formula I 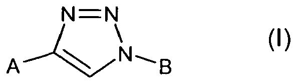 a stereoisomer or a mixture of its stereoisomers, or a pharmaceutically acceptable addition salt thereof, wherein
one of A and B represents a pyridinyl group optionally substituted one or more times with a substituent selected from halo, trifluoromethyl, trifluoromethoxy, cyano, nitro and alkoxy; and
the other one of A and B represents a pyridinyl group optionally substituted one or more times with a substituent selected from halo, trifluoromethyl, trifluoromethoxy, cyano and nitro.

2. The triazole derivative of claim 1, a stereoisomer or a mixture of its stereoisomers, or a pharmaceutically acceptable addition salt thereof, wherein
A represents pyridinyl substituted with halo.

3. The triazole derivative of claim 1, a stereoisomer or a mixture of its stereoisomers, or a pharmaceutically acceptable addition salt thereof, wherein
A represents pyridinyl substituted with alkoxy.

4. The triazole derivative of claim 1, a stereoisomer or a mixture of its stereoisomers, or a pharmaceutically acceptable addition salt thereof, wherein
A represents pyridinyl.

5. The triazole derivative of any one of claims 1-4, a stereoisomer or a mixture of its stereoisomers, or a pharmaceutically acceptable addition salt thereof, wherein
B represents a pyridinyl group substituted with halo.

6. The triazole derivative of any one of claims 1-4, a stereoisomer or a mixture of its stereoisomers, or a pharmaceutically acceptable addition salt thereof, wherein
B represents a pyridinyl group.

7. The triazole derivative compound of claim 1, which is
3-(1-Pyridin-3-yl-1*H*-[1,2,3]triazol-4-yl)-pyridine;
3-Fluoro-5-(1-pyridin-3-yl-1H-[1,2,3]triazol-4-yl)-pyridine;
2-Fluoro-5-(1-pyridin-3-yl-1H-[1,2,3]triazol-4-yl)-pyridine;
2-Methoxy-5-(1-pyridin-3-yl-1 H-[1,2,3]triazol-4-yl)-pyridine;
3-Fluoro-5-(4-pyridin-3-yl-[1,2,3]triazol-1-yl)-pyridine;
2-Fluoro-5-(4-pyridin-3-yl-[1,2,3]triazol-1-yl)-pyridine; or
3-Bromo-5-(4-pyridin-3-yl-[1,2,3]triazol-1-yl)-pyridine;
a stereoisomer or a mixture of its stereoisomers, or a pharmaceutically acceptable addition salt thereof.

8. A pharmaceutical composition comprising a therapeutically effective amount of a triazole derivative of any one of claims 1-7, a stereoisomer or a mixture of its stereoisomers, or a pharmaceutically acceptable addition salt thereof, together with at least one pharmaceutically acceptable carrier or diluent.

9. The triazole derivative of any one of claims 1-7, a stereoisomer or a mixture of its stereoisomers, or a pharmaceutically acceptable salt thereof, for use as a medicament.

10. Use of a triazole derivative of any one of claims 1-7, a stereoisomer or a mixture of its stereoisomers, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors.

11. The use according to claim 10, wherein the disease, disorder or condition is a cognitive disorder, learning deficit, memory deficits and dysfunction, Down's syndrome, Alzheimer's disease, attention deficit, attention deficit hyperactivity disorder (ADHD), Tourette's syndrome, psychosis, depression, bipolar disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, amyotrophic lateral sclerosis (ALS), anxiety, non-OCD anxiety disorders, convulsive disorders, convulsions, epilepsy, neurodegenerative disorders, transient anoxia, induced neuro-degeneration, neuropathy, diabetic neuropathy, peripheral dyslexia, tardive dyskinesia, hyperkinesia, pain, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to postherpetic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or abuse liability and withdrawal symptoms caused by termination of use of addictive substances, including nicotine containing products such as tobacco, opioids such as heroin, cocaine and morphine, cannabis, benzodiazepines and benzodiazepine-like drugs, and alcohol.

## Patentansprüche

1. Triazolderivat, das durch Formel I wiedergegeben ist 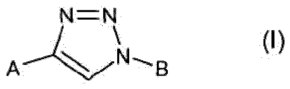 ein Stereoisomer oder eine Mischung von seinen Stereoisomeren, oder ein pharmazeutisch verträgliches Additionssalz davon, worin
eines von A und B eine Pyridinylgruppe bedeutet, die gegebenenfalls einfach oder mehrfach mit einem Substituenten substituiert ist, der ausgewählt ist aus Halogen, Trifluormethyl, Trifluormethoxy, Cyano, Nitro und Alkoxy; und
das andere von A und B eine Pyridinylgruppe bedeutet, die gegebenenfalls einfach oder mehrfach mit einem Substituenten substituiert ist, der ausgewählt ist aus Halogen, Trifluormethyl, Trifluormethoxy, Cyano und Nitro.

2. Triazolderivat nach Anspruch 1, ein Stereoisomer oder eine Mischung von seinen Stereoisomeren, oder ein pharmazeutisch verträgliches Additionssalz davon, worin A mit Halogen substituiertes Pyridinyl bedeutet.

3. Triazolderivat nach Anspruch 1, ein Stereoisomer oder eine Mischung von seinen Stereoisomeren, oder ein pharmazeutisch verträgliches Additionssalz davon, worin A mit Alkoxy substituiertes Pyridinyl bedeutet.

4. Triazolderivat nach Anspruch 1, ein Stereoisomer oder eine Mischung von seinen Stereoisomeren, oder ein pharmazeutisch verträgliches Additionssalz davon, worin A Pyridinyl bedeutet.

5. Triazolderivat nach einem der Ansprüche 1-4, ein Stereoisomer oder eine Mischung von seinen Stereoisomeren, oder ein pharmazeutisch verträgliches Additionssalz davon, worin B eine mit Halogen substituierte Pyridinylgruppe bedeutet.

6. Triazolderivat nach einem der Ansprüche 1-4, ein Stereoisomer oder eine Mischung von seinen Stereoisomeren, oder ein pharmazeutisch verträgliches Additionssalz davon, worin B eine Pyridinylgruppe bedeutet.

7. Triazolderivatverbindung nach Anspruch 1, welche
3-(1-Pyridin-3-yl-1*H*-[1,2,3]triazol-4-yl)-pyridin;
3-Fluor-5-(1-pyridin-3-yl-1 H-[1,2,3]triazol-4-yl)-pyridin;
2-Fluor-5-(1-pyridin-3-yl-1 H-[1,2,3]triazol-4-yl)-pyridin;
2-Methoxy-5-(1-pyridin-3-yl-1 H-[1,2,3]triazol-4-yl)-pyridin;
3-Fluor-5-(4-pyridin-3-yl-[1,2,3]triazol-1-yl)-pyridin;
2-Fluor-5-(4-pyridin-3-yl-[1,2,3]triazol-1-yl)-pyridin; oder 3-Brom-5-(4-pyridin-3-yl-[1,2,3]triazol-1-yl)-pyridin ist;
ein Stereoisomer oder eine Mischung von ihren Stereoisomeren, oder ein pharmazeutisch verträgliches Additionssalz davon.

8. Pharmazeutische Zusammensetzung umfassend eine therapeutisch wirksame Menge von einem Triazolderivat nach einem der Ansprüche 1-7, einem Stereoisomer oder einer Mischung von seinen Stereoisomeren, oder einem pharmazeutisch verträglichen Additionssalz davon, zusammen mit wenigstens einem pharmazeutisch verträglichen Träger oder Verdünnungsmittel.

9. Triazolderivat nach einem der Ansprüche 1-7, ein Stereoisomer oder eine Mischung von seinen Stereoisomeren, oder ein pharmazeutisch verträgliches Salz davon, zur Verwendung als ein Medikament.

10. Verwendung eines Triazolderivats nach einem der Ansprüche 1-7, eines Stereoisomers oder einer Mischung von seinen Stereoisomeren, oder eines pharmazeutisch verträglichen Additionssalzes davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei die Krankheit, die Störung oder der Zustand auf die Modulation von cholinergen Rezeptoren anspricht.

11. Verwendung nach Anspruch 10, wobei es sich bei der Krankheit, der Störung oder dem Zustand um eine kognitive Störung, ein Lerndefizit, Gedächtnisdefizite und eine Gedächtnisdysfunktion, das Down-Syndrom, die Alzheimer-Krankheit, ein Aufmerksamkeitsdefizit, eine Aufmerksamkeitsdefizit-Hyperaktivitätsstörung (ADHD), das Tourette-Syndrom, eine Psychose, eine Depression, eine bipolare Störung, eine Manie, eine manische Depression, eine Schizophrenie, kognitive Defizite oder Aufmerksamkeitsdefizite in Verbindung mit Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, Autismus, die Parkinson-Krankheit, Chorea Huntington, amyotrophe Lateralsklerose (ALS), Angst, Nicht-OCD-Angststörungen, konvulsive Störungen, Krämpfe, Epilepsie, neurodegenerative Störungen, vorübergehende Anoxie, induzierte Neurodegeneration, Neuropathie, diabetische Neuropathie, periphere Dyslexie, tardive Dyskinesie, Hyperkinesie, Schmerzen, milde Schmerzen, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, neuropathische Schmerzen, chronische Kopfschmerzen, zentrale Schmerzen, Schmerzen in Verbindung mit diabetischer Neuropathie, mit postherpetischer Neuralgie oder mit peripherer Nervenverletzung, Bulimie, das posttraumatische Syndrom, eine Sozialphobie, Schlafstörungen, eine Pseudodemenz, das Ganser-Syndrom, das prämenstruelle Syndrom, das Syndrom der späten Lutealphase, das chronische Ermüdungssyndrom, Mutismus, Trichotillomanie, Jetlag, Arrhythmien, Kontraktionen eines glatten Muskels, Angina pectoris, eine Frühgeburt, eine Diarrhö, Asthma, tardive Dyskinesie, Hyperkinesie, eine vorzeitige Ejakulation, Erektionsschwierigkeiten, Hochdruck, entzündliche Störungen, entzündliche Hautstörungen, Akne, Rosacea, Morbus Crohn, eine entzündliche Darmerkrankung, Colitis ulcerosa, eine Diarrhö oder eine Anfälligkeit für Missbrauch und Entzugssymptome, die durch die Beendigung des Gebrauchs von süchtigmachenden Substanzen, einschließlich nikotinhaltiger Produkte wie Tabak, Opioide wie Heroin, Kokain und Morphin, Cannabis, Benzodiazepine und Benzodiazepin-ähnlicher Drogen bzw. Arzneimittel, und Alkohol, verursacht sind, handelt.

## Revendications

1. Dérivé de triazole représenté par la formule I 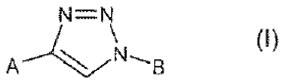 stéréoisomère ou mélange de ses stéréoisomères, ou sel d'addition pharmaceutiquement acceptable de celui-ci, dans lequel
l'un de A et B représente un groupe pyridinyle facultativement substitué une ou plusieurs fois par un substituant sélectionné parmi halogéno, trifluorométhyle, trifluorométhoxy, cyano, nitro et alcoxy ; et
l' autre de A et B représente un groupe pyridinyle facultativement substitué une ou plusieurs fois par un substituant sélectionné parmi halogéno, trifluorométhyle, trifluorométhoxy, cyano et nitro.

2. Dérivé de triazole selon la revendication 1, stéréoisomère ou mélange de ses stéréoisomères, ou sel d'addition pharmaceutiquement acceptable de celui-ci, dans lequel
A représente un pyridinyle substitué par un halogéno.

3. Dérivé de triazole selon la revendication 1, stéréoisomère ou mélange de ses stéréoisomères, ou sel d'addition pharmaceutiquement acceptable de celui-ci, dans lequel
A représente un pyridinyle substitué par un alcoxy.

4. Dérivé de triazole selon la revendication 1, stéréoisomère ou mélange de ses stéréoisomères, ou sel d'addition pharmaceutiquement acceptable de celui-ci, dans lequel
A représente un pyridinyle.

5. Dérivé de triazole selon l'une quelconque des revendications 1 à 4, stéréoisomère ou mélange de ses stéréoisomères, ou sel d'addition pharmaceutiquement acceptable de celui-ci, dans lequel
B représente un groupe pyridinyle substitué par un halogéno.

6. Dérivé de triazole selon l'une quelconque des revendications 1 à 4, stéréoisomère ou mélange de ses stéréoisomères, ou sel d'addition pharmaceutiquement acceptable de celui-ci, dans lequel
B représente un groupe pyridinyle.

7. Composé dérivé de triazole selon la revendication 1, qui est
la 3-(1-pyridin-3-yl-1*H*-[1,2,3]triazol-4-yl)-pyridine ;
la 3-fluoro-5-(1-pyridin-3-yl-1*H*-[1,2,3]triazol-4-yl)-pyridine ;
la 2-fluoro-5-(1-pyridin-3-yl-1*H*-[1,2,3]triazol-4-yl)-pyridine ;
la 2-méthoxy-5-(1-pyridin-3-yl-1*H*-[1,2,3]triazol-4-yl)-pyridine ;
la 3-fluoro-5-(4-pyridin-3-yl-1*H*-[1,2,3]triazol-1-yl)-pyridine ;
la 2-fluoro-5-(4-pyridin-3-yl-1*H*-[1,2,3]triazol-1-yl)-pyridine ; ou
la 3-bromo-5-(4-pyridin-3-yl-1*H*-[1,2,3]triazol-1-yl)-pyridine ;
un stéréoisomère ou un mélange de ses stéréoisomères, ou un sel d'addition pharmaceutiquement acceptable de celui-ci.

8. Composition pharmaceutique comprenant une quantité thérapeutiquement efficace d'un dérivé de triazole selon l'une quelconque des revendications 1 à 7, d'un stéréoisomère ou d'un mélange de ses stéréoisomères, ou d'un sel d'addition pharmaceutiquement acceptable de celui-ci, conjointement avec au moins un vecteur ou diluant pharmaceutiquement acceptable.

9. Dérivé de triazole selon l'une quelconque des revendications 1 à 7, stéréoisomère ou mélange de ses stéréoisomères, ou sel pharmaceutiquement acceptable de celui-ci, pour une utilisation en tant que médicament.

10. Utilisation d'un dérivé de triazole selon l'une quelconque des revendications 1 à 7, d'un stéréoisomère ou d'un mélange de ses stéréoisomères, ou d'un sel d'addition pharmaceutiquement acceptable de celui-ci, pour la fabrication d'une composition pharmaceutique/d'un médicament pour le traitement, la prévention ou le soulagement d'une maladie ou d'un trouble ou d'une affection d'un mammifère, incluant un être humain, laquelle maladie, lequel trouble ou laquelle affection réagit à une modulation des récepteurs cholinergiques.

11. Utilisation selon la revendication 10, dans laquelle la maladie, le trouble ou l'affection est un trouble cognitif, un défaut d'apprentissage, des déficits et un dysfonctionnement de la mémoire, un syndrome de Down, une maladie d'Alzheimer, un déficit de l'attention, un trouble de l'hyperactivité avec déficit de l'attention (THDA), un syndrome de Tourette, une psychose, une dépression, un trouble bipolaire, une manie, une maniaco-dépression, une schizophrénie, des déficits cognitifs ou de l'attention liés à une schizophrénie, des troubles obsessionnels compulsifs (TOC), des troubles paniques, des troubles de l'alimentation tels qu'une anorexie mentale, une boulimie et une obésité, une narcolepsie, une nociception, une démence associée au SIDA, une démence sénile, un autisme, une maladie de Parkinson, une maladie de Huntington, une sclérose latérale amyotrophique (SLA), une anxiété, des troubles de l'anxiété non TOC, des troubles convulsifs, des convulsions, une épilepsie, des troubles neurodégénératifs, une anoxie transitoire, une neurodégénération induite, une neuropathie, une neuropathie diabétique, une dyslexie périphérique, une dyskinésie tardive, une hyperkinésie, une douleur, une douleur légère, une douleur modérée ou sévère, une douleur à caractère aigu, chronique ou récurrent, une douleur provoquée par une migraine, une douleur postopératoire, une douleur du membre fantôme, une douleur inflammatoire, une douleur neuropathique, des céphalées chroniques, une douleur centrale, une douleur liée à une neuropathie diabétique, à une névralgie post-herpétique, ou à une lésion du nerf périphérique, une boulimie, un syndrome post-traumatique, une phobie sociale, des troubles du sommeil, une pseudodémence, un syndrome de Ganser, un syndrome prémenstruel, un syndrome de la phase lutéale tardive, un syndrome de fatigue chronique, un mutisme, une trichotillomanie, un décalage horaire, des arythmies, des contractions du muscle lisse, une angine de poitrine, un travail prématuré, une diarrhée, un asthme, une dyskinésie tardive, une hyperkinésie, une éjaculation précoce, une difficulté d'érection, une hypertension, des troubles inflammatoires, des troubles inflammatoires cutanés, une acné, une rosacée, une maladie de Crohn, une maladie intestinale inflammatoire, une colite ulcéreuse, une diarrhée ou des risques d'abus et des symptômes de sevrage provoqués par la fin de l'utilisation de substances addictives, incluant les produits contenant de la nicotine tels que le tabac, les opioïdes tels que l'héroïne, la cocaïne et la morphine, le cannabis, les benzodiazépines et les médicaments de type benzodiazépine, et l'alcool.
